Europäisches Patentamt

European Patent Office

Office européen des brevets

(11) Publication number: 0 272 098
A2

# (12) EUROPEAN PATENT APPLICATION

(21) Application number: 87311062.1

(22) Date of filing: 15.12.87

(51) Int. Cl.4: C12Q 1/68 , //C12Q1/70

(30) Priority: 15.12.86 US 941379

(43) Date of publication of application:
22.06.88 Bulletin 88/25

(84) Designated Contracting States:
AT BE CH DE ES FR GB GR IT LI LU NL SE

(71) Applicant: CITY OF HOPE NATIONAL MEDICAL CENTER
1500 East Duarte Road
Duarte California 91010(US)

(72) Inventor: Murakawa, George J.,
8611 Acacia Drive,
Cypress, California 91010,(US)
Inventor: Wallace, Bruce R.,
1010 Buena Vista
South Pasadena, California 91030,(US)
Inventor: Zaia, John A.,
300 Armada Drive,
Arcadia, California 91106(US)
Inventor: Rossi, John J.,
346 Cimmeron Trail,
Glendora, California 91740(US)

(74) Representative: De Minvielle-Devaux, Ian Benedict Peter et al
CARPMAELS & RANSFORD 43, Bloomsbury Square
London WC1A 2RA(GB)

(54) Method for amplification and detection of RNA sequences.

(57) A process for the rapid amplification and detection of a RNA sequence which comprises adding to RNA two converging oligodeoxyribonucleotide primers at least one of which is complementary to a sequence included in said RNA; thereafter extending at least one of said primers with one or both of reverse transcriptase and DNA polymerase I, determining whether said sequence is present in said amplified segment by use of synthetic oligodeoxyribonucleotide probe complementary to a region within the segment.

## METHOD FOR AMPLIFICATION AND DETECTION OF RNA SEQUENCES

Human immunodeficiency virus (HIV), or human T-lymphotropic virus (HTLVIII/LAV), has been determined as the etiological agent of acquired immune deficiency syndrome, AIDS.[1] HIV infects T-lymphocytes by recognizing the T4 antigen.[2] Brain cells and macrophages are also targets for viral infection.[3] The entire viral sequence has been determined, and is similar in structure to other retroviruses.[4] Between the long terminal repeats (LTRs) are the virally encoded gag, pol, and env genes. Additionally, at least three other genes which code for trans-acting regulatory protein have been identified.[5]

[1] Weiss, A., Hollander, H. and Stobo, J. Ann. Rev. Med. 36, 545-562 (1985); Wong-Staal, F. and Gallo, R.C., Nature 317, 395-403 (1985); Rabson, A.B. and Martin, M.A. Cell 40, 477-480 (1985).

[2] Dalgleish, A.B., et al. Nature 312 , 277-284 (1985); Maddon, P.J., et al, Cell 47 , 333-348 (1986); McDougal, J.S., et al, J. Immunol. 135, 3151-3162 (1985).

[3] Epstein, L.G., et at, AIDS Res. 1, 447-454 (1985); Ho, D.D., et al, N. Eng. J. Med. 313, 1498-1504 (1985); Koenig, S., et al. Science 233, 1089-1093 (1986); Levy, J.A., et al, Lancet 11, 586-588 (1985); Sharer, L.R., et al, Human Path. 17, 271-284 (1986); Shaw, G.M., et al, Science 227, 177-182.

[4] Ratner, L., et al, Nature 313, 277-284 (1985); Wain-Hobson, S., Sonigo, P., Danos, O., Cole, S., and Alizon, M. Cell 40, 9-17 (1985); Muesing, M.A., et al, Nature 313, 450-458 (1985); Sanchez-Pescador, R., et al, Science 227, 484-492 (1985).

[5] Sodroski, J., et al, Nature 321, 412-417 (1986); Rosen, C.A., et al, Nature 319, 555-559 (1986); Fischinger, P.J. and Bolognesi, D.P. in AIDS (ed. DeVita, Jr., V.T., Helman, S. and Rosenberg, S.A.) 55-58 (Lippincott Co., Philadelphia, PA, 1985).

Clinically, the rapid and practical detection of HIV infection depends on the presence of serum antibodies against HIV-specific antigens - the tests for which are not 100% reliable.[6] Furthermore, virus can only be isolated from about 80% of patients who test positive.[7] This invention provides a test to rapidly diagnose/confirm potential HIV positive patients.

[6] Groopman, J.E., et al, Blood 66, 742-744 (1985); Reed, K.C. and Mann, D.A. Nucelic Acids Res. 13, 7207-7221 (1985).

[7] Wong-Staal, F. and Gallo, R.C., Nature 317 , 395-403 (1985).

Recently, it was shown that small amounts of DNA samples, undetectable with standard nucleic acid hybridization methods can be specifically detected after amplification. This method makes use of repeated synthesis of nucleic acid sequences bound by oppositely oriented primers, and is referred to as the polymerase chain reaction, or PCR.[8] This technique is extended by this invention to accommodate the amplification of an RNA template.

[8] Saiki, R.K., et al, Science 230, 1350-1354 (1985).

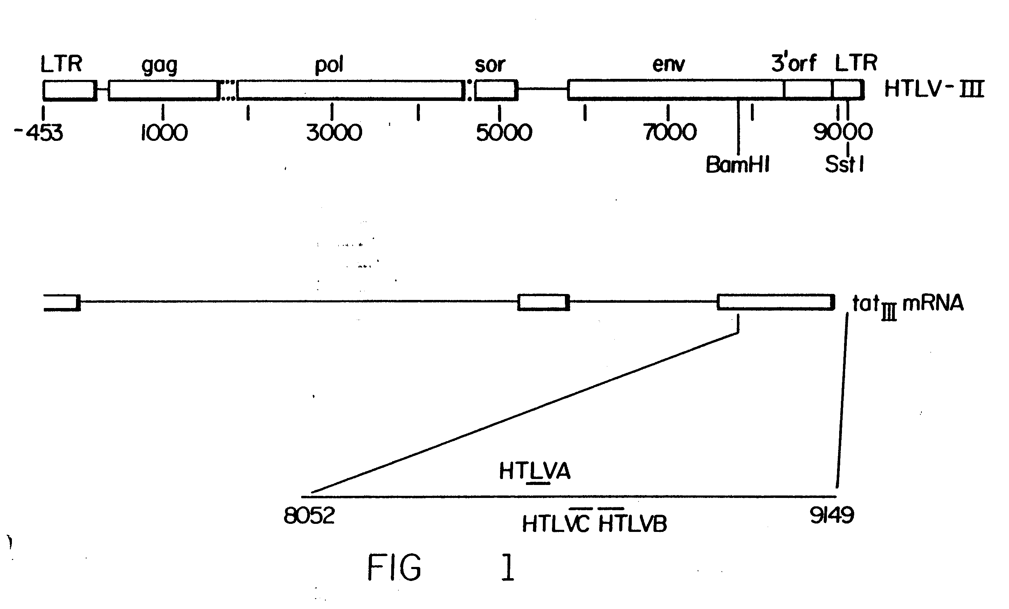

The distal portion of the HIV virus within the 3′ ORF region was chosen for amplification experiments because limited sequence polymorphism in this region among different viral isolates will, in the future, enable oligodeoxyribonucleotide hybridization discrimination between these variants.[9] Figure 1 describes the region of amplification and the oligodeoxyribonucleotide synthesized to amplify and probe for the virus.

[9] Ratner, L., et al, Nature 313, 277-284 (1985); Wain-Hobson, S., Sonigo, P., Danos, O., Cole, S., and Alizon, M. Cell 40, 9-17 (1985); Muesing, M.A., et al, Nature 313, 450-458 (1985); Sanchez-Pescador, R., eg al, Science 227, 484-492 (1985).

In Figure 1 the entire HTLV-III virus is shown. Genes and LTR's are represented by open boxes. The 1.1 kb⁻ BamHI-SstI fragment is enlarged and shown below the entire virus. This fragment contains the amplification region. HTLVA, 5′ATGCTGA-TTGTGCCTGGCTA, is a sense oligodeoxyribonucleotide, encoding nucleotides 8538 to 8547. HTLVB, 5′TGAATTAGCCCTTCCAGTCC, is an antisense oligodeoxyribonucleotide, encoding nucleotides 8677 to 8658. Oligodeoxyribonucleotide HTLVA and HTLVB were used as the primers for HIV amplification. HTLVC, 5′AAGT-GGCTAAGATCTACAGCTGCCT, is an antisense oligodeoxyribonucleotide, encoding nucleotides 8642 to 8618, and was used as the probe to detect amplification.

To perform these experiments, two converging oligodeoxyribonucleotide primers, oriented in opposite directions, were added to the RNA. The primer complementary to the viral RNA was annealed to the template and extended with reverse transcriptase. After completion of this reaction, samples were heated to 95°C to denature the complementary strands, annealed with both primers, and once again primer-extended. In early cycles (2-6), both reverse transcriptase and the large fragment of DNA polymerase I (Klenow) were added for elongation. Ribonuclease A was added (cycle 7) to remove the complexity of RNA and facilitate primer hybridization. All subsequent cycles were performed by the addition of only DNA polymerase 1. Amplified samples were tested by alkaline Southern blot analysis,[10] using as a probe a third synthetic oligodeoxyribonucleotide, complementary to a region within the amplified segment (see Fig. 1).

[10] Southern, E.M., J. Mol. Biol. 98, 503-517 (1975); Chirgwin, J., et al, Biochem. 18, 5294-5299 (1979).

Although DNA templates have been amplified successfully, use of an RNA template provides greater resolution. Since expression of messenger RNA in higher eukaryotic cells is selective and is often tissue specific or time dependent, amplification of RNA can rely on a less complex template. Treatment with ribonuclease A in later cycles also reduces the complexity of the template and facilitates specific primer annealing.

Preliminary experiments to determine whether the polymerase chain reaction could be applied to RNA templates indicated that RNAs transcribed in vitro from both pGM92 and pGM93 (Fig. 2), plasmids containing the 3' ORF region in both orientations, could be amplified with equal efficiency. To test the efficiency of amplification from pGM92 RNA, a progression of amplification rounds was performed.

The efficiency of amplification of in vitro synthesized RNA is illustrated by the use of the plasmid pSP64-BH10-R3 (Biotech Research Laboratories, Inc.), containing the entire HTLV-III virus excluding the LTRs, as the starting material for the following subclone vectors. A 1.1 kb BamHI restriction fragment including HTLVIII sequences 8052 to 9149 was subcloned in both orientations into the BamHI site of the transcription vector pGEM2 (Promega Biotec). The resulting plasmids, pGM92 (+ strand) and pGM93 (-strand), were digested with EcoR1 and transcribed with T7 RNA polymerase using a T7 transcription kit (BioRad Laboratories, Inc.). RNA from PGM92 was used in this experiment to test the efficiency of amplification. 10[1] pmol of RNA was subjected to 4, 5, 8 and 10 cycles of amplification. Amplification was performed using IX and amplification buffer (10 mM tris-HCl, pH 7.5; 10 mM MgCl$_2$; 66 mM NaCl; 1 mM dithiothreitol), 1.5 mM of each dNTP, and 1.0 uM of each oligodeoxyribonucleotide (HTLVA and HTLVB) in a final reaction volume of 100 ul. Samples were heated to 95°C for 2 minutes, spun in a microfuge for 5 seconds, cooled to 37°C for 2 minutes, at which time 1.0 ul of reverse transcriptase (2.0 units, BioRad), diluted in amplification buffer, was added for 2 minutes. Cycles 2-5 were performed as described above, except both reverse transcriptase and Klenow (0.5 units, Boehringer Mannheim) were added. In cycle 6. RNase A was added (0.45 ug) and only DNA pol I was used. All subsequent cycles of amplification were performed with only the presence of DNA Pol I. After completion of the last cycle of amplification, samples were placed on ice and a 10.0 ul portion was electrophoresed in a 1.8% agarose gel. The DNA was transferred to Zeta probe (BioRad) using an alkaline blotting procedure[11] and prehybridized and hybridized as follows: The prehybridization reaction was performed at 65°C for 1 to 3 hours in 20 ml of 6X SSPE (1.0 M NaCl, 0.06 M NaPO$_4$, 0.006 M EDTA); 1.0% SDS; 0.5% rehydrated, powder skim milk (Alba); and 10ug per ml of sonicated, denatured salmon sperm DNA. The hybridization reaction was in 20 ml of the same buffer, except the salmon sperm DNA was omitted and replaced with 20 pmol of 5'-$^{32}$p-labelled oligodeoxyribonucleotide HTLVC (ca. 3 x 10$^8$ cpm). Hybridization was for 1 hour to overnight at 65°C. The hybridized filter was washed with three 250 ml volumes of 6X SSC (0.95 M NaCl. 0.095 M Na Citrate), 0.1% SDS at 65°C for 5 minutes each, and autoradiographed at -70°C for 1 hour on Kodak XAR-5 film with an intensifying screen.

[11] Southern, E.M., J. Mol. Biol. 98, 503-517 (1975).

Densitometric scanning and integration amplification were performed. This revealed a 3.81 fold level of amplification. Thus, if 21 cycles were performed with this template, and since only one strand is synthesized during the first cycle, we calculate the theoretical amplification would be over 400,000 fold. In this same experiment, comparing 6.8 and 10 rounds, the amplification was no longer exponential. One explanation for this result is that the primer concentration significantly decreases and competition between reannealing synthesized strands and oligodeoxyribonucleotides continually increases.

To test the sensitivity of amplification, samples in which 10$^9$, 10$^7$, and 10$^5$ pmol of pGM92 RNA were used. After 21 cycles of replication, bands from each of the samples can be detected after Southern blot hybridization. The detection of the sample from only 10$^9$ pmol indicates that as few as 100 molecules of RNA are sufficient for detection after amplification.

Amplification of this RNA template is also possible in the presence of non-specific RNA. Using 10$^3$ pmol of GM92 RNA, 5.5 ug of bovine rRNA was added to the reaction mixture and specific amplification was seen at high efficiency.

RNA isolated from HIV infected cells can be efficiently utilized for amplification and detection. Polymerase chain reaction using only 10 ng of total RNA from HIV infected H9 cells was performed. A specific hybridizing band, about two orders of magnitude lower than the 1.0 ug sample, was observed. To test if the amplification of the in vivo sample was from RNA or residual DNA contamination, a control sample in which RNase A was added prior to amplification was examined. In this experiment, no hybridization band was detected after prolonged autoradiographic exposure.

Testing of the effects of anti-viral drugs on HIV viral proliferation has shown that oligodeoxymethylphosphates, complementary to specific regions of the virus, dramatically reduce virus production. Thus, the modified PCR technique will be useful for clinical assays of the efficacy of anti-HIV drugs.

Under certain conditions, this invention is useful to amplify and detect as few as 100 molecules of RNA. Most importantly, it can be used to detect HIV RNA in samples from 10 ng of total cellular RNA, which is comparable to ca. 100 to 1000 cells. From these experimental data, a reasonable estimate is that about 10 copies of HIV transcripts are present in each cell. This technique can be performed in less than one day. Its practical application to clinical diagnosis of AIDS-infected patients is thus apparent. Patients who harbor the AIDS viral genome but are not yet producing anti-AIDS antibodies might be diagnosed as uninfected by current screening methods. The present technique will enable detection of viral transcripts which may accumulate in the absence of viral protein translation during the early stages of infection.

## Claims

1. A process for the rapid amplification and detection of a RNA sequence which comprises adding to RNA two converging oligodeoxyribonucleotide primers at least one of which is complementary to a sequence included in said RNA;
thereafter extending at least one of said primers with one or both of reverse transcriptase and DNA polymerase I, determining whether said sequence is present in said amplified segment by use of a synthetic oligodeoxyribonucleotide probe complementary to a region within the segment.

0 272 098

LTR   gag        pol              sor        env          3'orf  LTR

|   |   |   |   |   |   |   |   HTLV-III

-453   1000      3000        5000      7000        9000

BamHI            SstI

tat_III mRNA

HTLVA

8052                                                    9149

HTLVC   HTLVB

FIG   1